(19) Europäisches Patentamt
European Patent Office
Office européen des brevets

(11) EP 1 663 997 B1

(12) **EUROPÄISCHE PATENTSCHRIFT**

(45) Veröffentlichungstag und Bekanntmachung des Hinweises auf die Patenterteilung:
**21.11.2012 Patentblatt 2012/47**

(21) Anmeldenummer: **04763137.9**

(22) Anmeldetag: **08.07.2004**

(51) Int Cl.:
***C07D 249/08*** (2006.01) ***C07D 231/16*** (2006.01)
***C07D 231/12*** (2006.01) ***A01N 43/56*** (2006.01)
***A01N 43/653*** (2006.01)

(86) Internationale Anmeldenummer:
**PCT/EP2004/007528**

(87) Internationale Veröffentlichungsnummer:
**WO 2005/035509 (21.04.2005 Gazette 2005/16)**

(54) **1H-AZOLYL-METHYL-AMIDE, VERFAHREN ZU IHRER HERSTELLUNG UND IHRE VERWENDUNG ALS NITRIFIKATIONSINHIBITOREN**

1H-AZOLYL-METHYL-AMIDES, METHOD FOR THE PRODUCTION THEREOF AND USE THEREOF AS NITRIFICATION INHIBITORS

1H-AZOLYL-METHYL-AMIDES, LEUR PROCEDE DE PREPARATION ET LEUR UTILISATION COMME INHIBITEURS DE NITRIFICATION

(84) Benannte Vertragsstaaten:
**AT BE BG CH CY CZ DE DK EE ES FI FR GB GR HU IE IT LI LU MC NL PL PT RO SE SI SK TR**

(30) Priorität: **18.09.2003 DE 10343277**

(43) Veröffentlichungstag der Anmeldung:
**07.06.2006 Patentblatt 2006/23**

(73) Patentinhaber: **SKW STICKSTOFFWERKE PIESTERITZ GmbH**
**06886 Lutherstadt Wittenberg (DE)**

(72) Erfinder:
- **RADICS, Ute**
  **06888 Dabrun (DE)**
- **NICLAS, Hans-Joachim**
  **12435 Berlin (DE)**
- **MICHEL, Hans-Jürgen**
  **04827 Machern (DE)**
- **WOZNIAK, Hartmut**
  **04451 Cunnersdorf (DE)**

(74) Vertreter: **Schneider, Michael et al**
**Heisse Kursawe Eversheds**
**Rechtsanwälte Partnerschaft**
**Maximiliansplatz 5**
**80333 München (DE)**

(56) Entgegenhaltungen:
**EP-A- 0 006 542** **EP-A- 0 012 216**
**EP-A- 1 342 412** **DE-A- 2 648 008**
**DE-A- 2 742 583** **SU-A- 666 174**

- **DATABASE CHEMCATS CHEMICAL ABSTRACTS SERVICE,COLUMBUS,OHIO,US; XP002307753 & 23. April 2003 (2003-04-23), ASINEX,ASINEX EXPRESS GOLD COLLECTION , 6 SCHUKINSKAYA STREET, MOSCOW, 123182, RUSSIA**
- **KATRITZKY, ALAN R. ET AL: "Isomerizations of N-(.alpha.-aminoalkyl)-1,2,4-triazoles and N-(.alpha.-aminoalkyl)tetrazoles" TETRAHEDRON ,46(2), 633-40 CODEN: TETRAB; ISSN: 0040-4020, 1990, XP002307752**

EP 1 663 997 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft N-(1H-Azolyl-methyl)amide, Verfahren zu ihrer Herstellung, Düngemittelzusammensetzungen, die sie aufweisen, sowie ihre Verwendung zur Hemmung bzw. Steuerung der Nitrifikation.

Die Anwendung von Stickstoffdüngemitteln, die Nitrifikationsinhibitoren (im folgenden auch als Nitrifikationshemmer bezeichnet) enthalten, ist dadurch besonders vorteilhaft, weil die im allgemeinen rasch ablaufende Nitrifikation, d. h. die mikrobielle Umwandlung von Amid- und Ammoniumstickstoff zu Nitratstickstoff, durch die Gegenwart derartig wirkender Substanzen auf diese Weise verzögert wird. Durch die so gesteuerte Nitratbildung im Boden wird eine zu intensive Nitratbildung und damit die Akkumulation von Nitrat vermieden. Eine so bewirkte Zurückdrängung des sonst entstehenden Nitratüberschusses im Boden konserviert den Düngerstickstoff in Form von Ammoniumionen, sorgt damit für eine höhere Düngereffizienz und führt gleichzeitig zu einer erheblich verringerten Umweltbelastung im Rahmen einer Stickstoffdüngung.

So ist bekannt, dass Nitrat im Gegensatz zum Ammonium im Boden nur äußerst schlecht sorbiert wird und demzufolge der Auswaschung bzw. der Verlagerung in tiefere Bodenschichten unterliegt. Andererseits führt die infolge einer Nitratanreicherung im Boden verstärkt einsetzende Denitrifikation zu gasförmigen Stickstoffverlusten, wobei neben molekularem Stickstoff auch solche Oxide wie Stickstoffmonoxid bzw. Lachgas emittiert werden, die zur Versauerung des Bodens beitragen bzw. als klimaverändernde Gase bekannt sind.

Durch die Hemmung bzw. Steuerung der Nitrifikation wird gleichzeitig einer Nitratanreicherung im Grundwasser entgegengewirkt.

Gerade in der Reduzierung der beschriebenen Verlustpotentiale liegt der wirtschaftliche Vorteil von mit Nitrifikationsinhibitoren versehenen Stickstoffdüngemitteln, der sich u. a. in der erhöhten Nährstoffeffizienz dieser Dünger niederschlägt. Um das zu unterstreichen sei erwähnt, dass N-Verluste von konventionellen Stickstoffdüngern durchaus unter ungünstigen Bedingungen Größenordnungen von bis zu 40 % und mehr annehmen können.

Als wirksame Nitrifikationshemmer wurden unter anderem stickstoffhaltige heterocyclische Verbindungen, bevorzugt aus der Reihe der 1H-Pyrazole und der 1,2,4-Triazole, vorgeschlagen, z. B. in US 3,635,690, US 4,969,946, US 4,523,940, EP-A 166 420, JP-OS 71-04135, US 3,697,244.

Ein wesentlicher Nachteil der meistens sehr gut nitrifikationshemmend wirkenden Pyrazolderivate besteht in ihrer relativ hohen Flüchtigkeit bzw. besonders bei am N-1-Stickstoffatom substituierten Verbindungen in ihrer Hydrolyseempfindlichkeit, in deren Folge zwar noch wirksame, aber wieder flüchtige Derivate gebildet werden. Aus diesen Gründen lässt sich eine Anwendung bei festen Düngemitteln, insbesondere in Kombinationen mit Harnstoff, nicht ohne weiteres realisieren. Zur Beseitigung dieses Mangels sind Vorschläge zur chemischen Abwandlung der 1-N-unsubstituierten Basispyrazole bekannt (DE-OS 27 45 833, EP-A 508 191, DD 292 232, DE-OS 40 18 395, EP-A 160 804, DE-OS 37 04 359, SU 1 470 737, DE-OS 44 46 194, EP-A 808 297). Mit dem Rückgang der Flüchtigkeit durch Derivatisierung ist jedoch in der Regel zugleich auch ein Abfall in der Wirkung als Nitrifikationsinhibitor verbunden. Außerdem sind solche Herangehensweisen mit einem Mehraufwand an Syntheseschritten gekoppelt, die fast durchgängig zu einer Kostenbelastung führen, so dass ihre Anwendung ökonomisch nicht mehr sinnvoll ist.

Andere Lösungen zur Reduzierung der Flüchtigkeit von nitrifikationshemmend wirkenden Pyrazolen bestehen in der Ausnutzung des eigentlich bekannten Prinzips der Salzbildung mit Säuren (EP-A 529 473, WO 98/05 607, DE-OS 4018 395).

Sowohl die mineralsauren Salze der nitrifiziden Basispyrazole als auch nitrifizid wirkende Pyrazole, die am 1-N-Stickstoff hydrophile Gruppen tragen, können als Nitrifikationsinhibitoren noch nicht voll befriedigen, da sie eine zu geringe Hydrolysestabilität besitzen, wodurch die Wirkungsdauer im Boden und die Lagerstabilität herabgesetzt ist. Dies betrifft z. B. N-Glyoxylpyrazole (DE-A 37 04 359) oder N-Hydroxymethylpyrazole (SU-A 1 470 737). Mit der Einführung von verschiedenartigen lipophilen Gruppen in 1-N-Position (DE-OS 101 17 821 A 1, DE-OS 101 35 423 A 1) konnte zwar die Hydrolyseanfälligkeit dieser Pyrazolderivate beseitigt werden, jedoch sind diese Nitrifikationsinhibitoren bei niedrigen Konzentrationen nicht mehr ausreichend lang leistungsfähig. Ein weiterer Mangel besteht in den aufwendigen Herstellungsverfahren für diese Wirkstoffe.

(1H-Azolyl-methyl)amide sind offenbart in Database Chemcats Chemical Abstracts, XP002307753, EP-A-1 342 412, DE 26 48 008 A, EP-A-0 012 216, EP-A-0 006 542, DE 27 42 583 A, SU 666 174 A, und Katritzky et al, Tetrahedron 46(2), 633-40, 1990.

Der vorliegenden Erfindung lag die Aufgabe zugrunde, Wirkstoffe zur Hemmung bzw. Steuerung der Nitrifikation von Ammoniumstickstoff zu entwickeln, die aufgrund ihrer geringen Flüchtigkeit und ihrer Hydrolysestabilität alle Applikationsverfahren, aber auch alle Möglichkeiten zur Einarbeitung in oder Aufbringung auf mineralische stickstoffhaltige Dünger zulassen und außerdem eine ausgezeichnete Residualwirkung aufweisen und unabhängig davon auch eine Anwendung zu organischen Düngern zulassen. Gleichzeitig sollten diese Wirkstoffe durch eine unkomplizierte, ökonomisch sinnvolle Synthese herstellbar sein.

Diese Aufgabe wurde erfindungsgemäß durch die N-(1H-Azolyl-methyl)amide entsprechend Formel I gelöst.

Es hat sich nämlich gezeigt, dass diese Verbindungen neben der sehr guten nitrifikationshemmenden Wirkung

den außerordentlichen Vorteil einer sehr geringen Hydrolyseneigung besitzen, wodurch Wirkstoffverluste bei der Oberflächenapplikation, aber auch bei der Lagerung von Dünger-Wirkstoff-Gemischen nahezu ausgeschlossen sind. Im Gegensatz zu bereits bekannten Azolderivaten liegt der besondere Vorteil der erfindungsgemäßen Verbindungen darin, dass sie nicht nur problemlos mit vielen »nitrathaltigen« N- Düngemitteln wie Ammonsulfatsalpeter und sauer reagierenden Düngern wie beispielsweise Ammoniumsulfat kombiniert werden können, ohne dass sie vorher in ihre Salze überführt werden müssen, um Wirkstoffverluste infolge Flüchtigkeit zu verhindern, sondern aufgrund der beschriebenen Eigenschaften besonders verlustfrei mit harnstoffbasierten Düngemitteln formuliert werden können. Dies ist umso bemerkenswerter als bekannt ist, dass Harnstoff stets mit geringen Anteilen von freiem Ammoniak begleitet ist, der die sonst schwächer basischen Azolderivate aus ihrem für die Wirkung unerlässlichen Verbund mit Harnstoff verdrängt und somit zu unterkritischen Konzentrationen an Nitrifikationsinhibitor führt, die eine ausreichende Nitrifikationshemmung nicht gewährleistet.

Außerdem sind die erfindungsgemäßen 1H-Azoyl-methyl-amide durch ihre Lipophilie und dem damit verbundenen hydrophoben Charakter für eine Aufbringung auf die Düngeroberfläche sehr gut geeignet

Überraschenderweise kommt die nitrifizide Wirkung der erfindungsgemäßen N-(1H-Azolyl-methyl)amide auch bei sehr geringen Wirkstoffkonzentrationen zur vollen Entfaltung, was nicht vorhersehbar war. So müssen beispielsweise schwerflüchtige Alkyl-1H-1-pyrazolylmethanamin-Derivate (DE 198 22 340 A1) in der Regel mit der doppelten Aufwandmenge eingesetzt werden, um die gleiche nitrifikationshemmende Wirkung wie die erfindungsgemäßen N-(1H-Azolylmethyl)amide zu erzielen.

Die erfindungsgemäß als Mittel Zur Hemmung bzw. Steuerung der Nitrifikation (verwendeten N-(1H-Azolylmethyl)amide entsprechen der allgemeinen Formel (I)

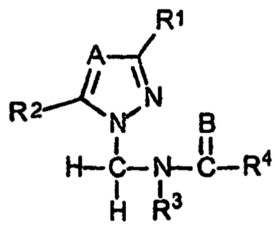

wobei A = CH, C-Halogen, C-$C_1$-$C_4$-Alkyl oder C-$C_3$-$C_8$-Cycloalkyl oder N ist,
$R^1$ und $R^2$ unabhängig voneinander für H, $C_1$-$C_4$-Alkyl oder $C_3$-$C_8$-Cycloalkyl seht
$R^3$ für $C_1$-$C_4$-Akyl, $C_3$-$C_8$-Cycloalkyl oder $C_6$-$C_{10}$-Aryl oder H, steht
$R^4$ für $C_1$-$C_{20}$-Alkyl, $C_3$-$C_8$-Cycloalkyl, $C_6$-$C_{10}$-Aryl oder Arylalkyl mit $C_1$-$C_4$-Alkyl- und $C_6$-$C_{10}$-Arylgruppen, wobei die Arylreste von $R^4$ gegebenenfalls einfach oder mehrfach mit $C_1$-$C_4$-Alkylsulfonyl, $C_1$-$C_4$-Alkoxy, $C_1$-$C_4$-Acyl, Halogen, Hydroxyl, Cyano, Nitro, Carboxyl oder $C_1$-$C_5$-Carboxyalkyl substituiert sein können, oder für $NHR^3$
oder für

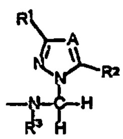

mit den für $R^1$, $R^2$, $R^3$ und A genannten Resten steht,
oder wobei $R^3$ und $R^4$ gemeinsam eine Ethylenbrücke bilden können für Sauerstoff oder Schwefel steht.

Die Erfindung betrifft ferner Verfahren zur Herstellung der erfindungsgemäßen N-(1H-Azolyl-methyl)amide, wie in den Ansprüchen 4 und 9 definiert, die in Anspruch 12 definierte Verwendung der N-(1H-Azolyl-methyl)amide sowie Zusammensetzungen, die diese N-(1H-Azolyl-methyl)amide enthalten, wie in Anspruch 13 definiert.

Weitere vorteilhafte und/oder bevorzugte Ausführungsformen der Erfindung sind Gegenstand der Unteransprüche.

Vorzugsweise stellt $R^1$ einen Methylrest und A = $CCH_3$ oder $R^1$ Methyl und A = CH oder CCl dar, und außerdem ist unabhängig davon bei einer weiteren Ausführungsform $R^2$ vorzugsweise H.

Der Begriff »Alkyl« bezieht sich hier, und zwar in jeder Kombination mit beliebigen anderen Gruppen, insbesondere (sofern nicht anders definiert) auf eine Alkyl-Gruppe, die 1 bis 8 Kohlenstoffatome aufweist, z.B. eine Methyl-, Ethyl-, n-Propyl-, Isopropyl-, n-Butyl-, Isobutyl-, tert-Butyl, Amyl, Isoamyl, n-Hexyl-, 2,2-Dimethylbutyl- oder n-Octyl-Gruppe. Im Folgenden wird zwar zur Vermeidung unnötiger Redundanz und der Einfachheit halber nur der Begriff »Alkyl-Gruppe« oder »Cycloalkyl-Gruppe« etc. verwendet, jedoch sollen jeweils die entsprechenden ungesättigten Gruppen

umfasst sein. Dem Fachmann ist klar, daß Alkenyl- öder Alkinyl-Gruppen mindestens 2 Kohlenstoffatome und cyclische Kohlenwasserstoff-Gruppen mindestens 3 Kohlenstoffatome aufweisen müssen. Die Alkyl-, Alkenyl- oder Alkinyl-Gruppen mit der entsprechenden Kohlenstoffanzahl können geradkettig oder verzweigt und einfach oder mehrfach ungesättigt sein.

Der Begriff »Aryl« bezieht sich auf eine aromatische cyclische, ggf. verzweigte Gruppe, die einen oder mehrere Ringe aufweist, und durch ein Gerüst gebildet wird, das 6 bis 10 Kohlenstoffatome enthält. Selbstverständlich können im Falle von mehreren Ringen einer der Ringe oder auch mehrere Ringe ganz oder teilweise hydriert sein (ein Beispiel dafür ist die 1,2,3,4,-Tetrahydro-naphthalen-1-yl-Gruppe).

Der Begriff »Arylalkyl« bezieht sich auf Gruppen, die entsprechend den obigen Definition sowohl Aryl- wie auch Alkyl-Gruppen und/oder Cyclo-Gruppen (weiter unten definiert) umfassen, z.B. eine Benzyl-Gruppe. Der Begriffe »Arylalkyl« soll zur Vermeidung unnötiger Redundanz auch den Begriff »Alkylaryl« umfassen.

Der Begriff »Cycloalkyl« bezieht sich auf eine gesättigte cyclische, ggf. verzweigte Gruppe, die einen oder mehrere Ringe aufweist, die ein Gerüst bilden, welches 3 bis 8 Kohlenstoffatome enthält, z.B. eine Cyclopropyl-, Cyclobutyl-, Cyclopentyl-, Cyclohexyl-Gruppe.

Der Begriff »Halogen« umfasst stets Fluor, Chlor, Brom und Iod.

Beispiele für Alkoxy-Gruppen bzw. Alkyloxy-Gruppen sind Methoxy-, Ethoxy-, Propoxy-, iso-Propoxy-, Butoxy oder tert.-Butoxy-Gruppen.

Beispiele für Acyl-Gruppen sind Acetyl-, Propionyl- oder Butyryl-Gruppen. oder Carboxybutyl-Gruppen.

Beispiele für Carboxyalkyl-Gruppen sind Carboxymethyl-, Carboxyethyl-, Carboxypropyl-

Ein weiterer Gegenstand der vorliegenden Erfindung ist die Verwendung der erfindungsgemäßen N-(1H-Azolylmethyl)amide zur Hemmung bzw. Regelung der Nitrifikation, wobei die erfindungsgemäß vorgeschlagenen Verbindungen in bekannter Weise als Nitrifikationsinhibitoren mit festen ammonium- und/oder amidhaltigen Düngemitteln kombinierbar sind. Als Stickstoffdüngemittel werden vorzugsweise Ammoniumsalze, wie z. B. Kalkammonsalpeter, Ammonsulfatsalpeter, Ammoniumnitrat, Amminiumsulfat oder Harnstoff eingesetzt. Möglich ist auch der Einsatz der erfindungsgemäßen N-(1H-Azolylmethyl)amide in Kombination mit organischen und flüssigen Düngemitteln. Aufwandmengen von 0,01 bis 10,0 Gew.-%, z. B. 0,1 bis 5,0 Gewichtsprozent, insbesondere 0,1 bis 3,0 Gewichtsprozent, berechnet auf den reduzierten Stickstoffgehalt des Düngers, d. h. auf Ammonium- und Carbamid-Stickstoff, genügen für den praktischen Einsatz. Selbstverständlich können die erfindungsgemäßen N-(1H-Azolyl-methyl)amide gegebenenfalls mit weiteren üblichen Nitrifikationsinhibitoren in Kombination verwendet werden. Wirkstoffformulierungen, Lösungen und Konzentrate, welche die erfindungsgemäßen N-(1H-Azolyl-methyl)amide aufweisen, sind ein weiterer Gegenstand der Erfindung.

Die Art und Weise, wie die erfindungsgemäß vorgeschlagenen Nitrifikationsinhibitoren mit dem Düngemittel kombiniert werden, unterliegt keinen besonderen Beschränkungen. Es eignen sich sämtliche im Stand der Technik bekannten Verfahren. So können die N-(1H-Azolyl-methyl)amide bevorzugt allein oder im Gemisch mit anderen Zusatzstoffen als Lösung oder gemeinsam mit einem geeigneten Konditionierungsmittel, beispielsweise mit einem hydrophobierend wirksamen paraffinischen Konditionierungsmittel auf die Oberfläche der Düngemittelgranulate aufgebracht werden, wobei das Lösemittel (vorzugsweise aprotische Lösemittel, wie z. B. Ester, Öle, Ketone, Ether etc.) z. B. durch Trocknung entfernt wird.

Alternativ hierzu können die N-(1H-Azolyl-methyl)amide allein oder im Gemisch mit anderen Zusatzstoffen zur homogenen Verteilung im Dünger in die Düngemittelschmelze oder slurry während des Formgebungsprozesses, z. B. zur Herstellung von Granulat, eingebracht werden. Flüssigen und organischen Düngern werden die erfindungsgemäßen Verbindungen vorzugsweise in Form einer geeigneten Formulierung zugegeben.

Es ist klar, das zur Formulierung übliche und dem Fachmann bekannte Hilfs-, Träger- und Streckstoffe oder eine Kombination dieser Mittel, oder auch oberflächenaktive Mittel, z. B. ein Benetzungs-, Dispersions-, Emulgierungs- oder Suspensionsmittel, verwendet werden können. Die jeweiligen Formulierungsverfahren entsprechen dem Stand der Technik und sind dem Fachmann bekannt.

Die erfindungsgemäßen Verbindungen zeichnen sich durch ihren lipophilen Charakter und durch ausgezeichnete nitrifizide Eigenschaften auch bei geringen Aufwandmengen aus. Die Einführung des Restes -C(B)$R^4$ führt zu einem erheblichen Anstieg der Hydrolysestabilität der Verbindungen und zu einer Verstärkung ihrer nitrifiziden Wirkung. Aufgrund dieser Eigenschaften sind die erfindungsgemäßen N-(1H-Azolyl-methyl)amide bevorzugt für eine Oberflächenapplikation auf den Düngemittelgranulaten geeignet

Ein weiterer Gegenstand der vorliegenden Erfindung sind N-(1H-Azolyl-methyl)amide der allgemeinen Formel (I)

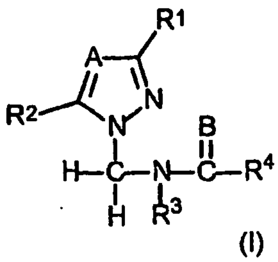

(I)

wobei A = CH, C-Halogen, C-$C_1$-$C_4$-Alkyl oder C-$C_3$-$C_8$-Cycloalkyl oder N ist,
$R^1$ und $R^2$ unabhängig voneinander für H, $C_1$-$C_4$-Alkyl oder $C_3$-$C_8$-Cycloalkyl steht
$R^3$ für $C_1$-$C_4$-Alkyl, $C_3$-$C_8$-Cycloalkyl oder $C_6$-$C_{10}$-Aryl oder H, steht
$R^4$ für $C_1$-$C_{20}$-Alkyl, $C_3$-$C_8$-Cycloalkyl, $C_6$-$C_{10}$-Aryl oder Arylalkyl mit $C_1$-$C_4$-Alkyl- und $C_6$-$C_{10}$-Arylgruppen, wobei die Arylreste von $R^4$ gegebenenfalls einfach oder mehrfach mit $C_1$-$C_4$-Alkylsulfonyl, $C_1$-$C_4$-Alkoxy, $C_1$-$C_4$-Acyl, Halogen, Hydroxyl, Cyano, Nitro, Carboxyl oder $C_1$-$C_5$-Carboxyalkyl substituiert sein können, oder für $NHR^3$
oder für

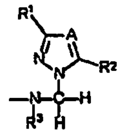

mit den für $R^1$, $R^2$, $R^3$ und A genannten Resten steht,
oder wobei $R^3$ und $R^4$ gemeinsam eine Ethylenbrücke bilden können
und B für Sauerstoff oder Schwefel steht, wobei A nicht N sein darf, wenn $R^1$, $R^2$ und $R^3$ für H und $R^4$ gleichzeitig für Phenyl oder für -CH=CH-Phenyl steht, A nicht CH sein darf, wenn $R^1$ für Methyl, $R^2$ und $R^3$ für H, B für Schwefel und

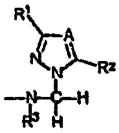

$R^4$ für

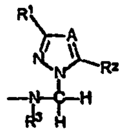

steht, und Anicht Nzeindarf, wenn $R^1$, $R^2$ für Methyl, $R^3$ für H, B für O und $R^4$ gleizhzeitig für mit Halogen Substituieten Phenyl steht.

Die $C_1$- bis $C_4$-Alkylreste $R^1$, $R^2$, $R^3$ und $R^4$ können linear oder verzweigt sein oder einen Cyclopropyl- oder Cyclobutylrest darstellen. Vorzugsweise stellt $R^1$ einen Methylrest und A = $CCH_3$ oder $R^1$ Methyl und A = CH oder CCl dar.

Die erfindungsgemäß vorgeschlagenen N-(1H-Azolyl-methyl)amide (I) (wobei $R^1$, $R^2$, $R^3$, $R^4$ sowie A und B die oben genannte Bedeutung besitzen) sind durch eine einfache Dreikomponentenreaktion gemäß der allgemeinen Gleichung (1) zugänglich. Dazu werden die Reaktionspartner Azol II, Formaldehyd III (z. B. als Paraformaldehyd) und das Amid IV (wobei $R^1$, $R^2$, $R^3$, $R^4$ sowie A und B die oben genannte Bedeutung besitzen) z. B. im (wobei $R^1$, $R^2$, $R^3$, $R^4$ sowie A und B die oben genannte Bedeutung besitzen) z. B. im Molverhältnis 1 : 1 : 1 bis 2,5 : 3 : 1 innig miteinander gemischt und z. B.15 bis 75 Minuten auf z. B. 100 bis 200 °C, vorzugsweise auf 120 bis 145 °C, z. B. auch ohne Lösungsmittel in der Schmelze, erhitzt.

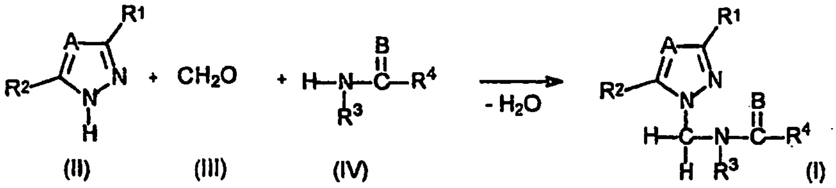

(1)

Die Umsetzung gemäß der allgemeinen Gleichung (1) kann auch in wässrigen Systemen, gegebenenfalls unter Zusatz von Lösungsvermittlern wie Alkoholen, beispielsweise Methanol oder Ethanol oder auch Acetonitril, unter Einsatz von wässriger Formaldehydlösung durchgeführt werden. Beispielsweise kann man die Reaktion bei Temperaturen von z. B. 40 bis 110 °C in beispielsweise Wasser als Lösungsmittel und mit einem Molverhältnis von Azol II zu Formaldehyd und Amid IV von z. B.1 : 1 : 1 bis 2,5 : 3 : 1 durchführen.

N-(1H-Azolyl-methyl)amide (I) (wobei $R^1$, $R^2$, $R^3$, $R^4$ sowie A und B die oben genannte Bedeutung besitzen) können auch erhalten werden, indem man die entsprechenden Hydroxymethyl-azole (V) (wobei $R^1$, $R^2$ und A die oben genannte Bedeutung besitzen) mit Amiden (IV) (wobei $R^3$, $R^4$ und B die oben genannte Bedeutung besitzen) gemäß der allgemeinen Gleichung (2) umsetzt, wobei das entsprechende Hydroxymethyl-azol (V) mit dem Amid (IV) z. B. im Molverhältnis 1 : 1,0 bis 2,0, gegebenenfalls unter Zusatz von z. B. 0,2 bis 10 % einer geeigneten Säure, vorzugsweise eines saueren Ionenaustauschers, in einem organischen Lösungsmittel, ausgewählt aus der Gruppe aromatische Kohlenwasserstoffe (wie z. B. Toluol), der Gruppe der Halogenkohlenwasserstoffe (wie z. B. Methylenchlorid) oder der Gruppe der Perfluorkohlenwasserstoffe (wie z. B. Perfluoroctan (PF5080)) oder ohne Lösungsmittel, bei Temperaturen von z. B. 40 bis 160 °C unter Wasserabscheidung zur Reaktion gebracht wird.

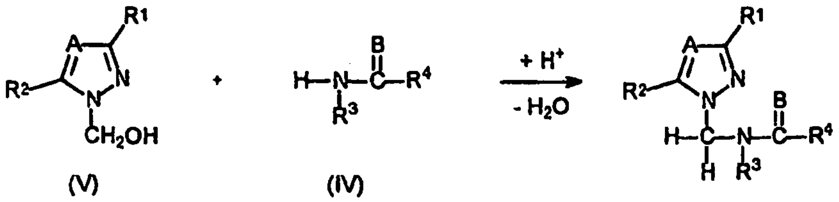


(2)

Die Hydroxymethyl-azole (V) bzw. deren Herstellung sind aus der Literatur bekannt (J. Hüttel Chem. Ber. 85 (1952) 820, OS 28 35 158, OS 199 13 475, L. Maier Phosphorus Sulfur 33 (1987) 41).

Die Reaktionsprodukte fallen als kristalline Substanzen an. Bei der Synthese der erfindungsgemäßen Verbindungen entsprechend den Gleichungen (1) und (2) wurde das Auftreten von Isomeren in Bezug auf die Stellung des $R^1$- und $R^2$-Substituenten außer Acht gelassen, es zeigt sich jedoch gelegentlich durch etwas breitere Schmelzbereiche. Falls gewünscht, können die jeweiligen Isomeren mittels der üblichen, dem Fachmann bekannten Verfahren getrennt werden.

Die nachfolgenden Beispiele sollen die Erfindung ohne Beschränkung näher veranschaulichen.

Beispiele

<u>Darstellung von N-(1H-Azolyl-methyl)amiden (I)</u>

Variante A:

Allgemeine Synthesevorschrift:

0,25 mol Azol (II), 0,25 mol Paraformaldehyd und 0,25 mol Amid (IV) werden innig miteinander vermischt. Man erhitzt vorsichtig bis auf 140 °C und rührt die klare Schmelze 15 bis 75 Minuten bei dieser Temperatur. Man lässt das Reaktionsgemisch erstarren und kristallisiert beispielsweise aus Essigsäureethylester oder Wasser um. Gegebenenfalls muss das Rohprodukt mit Bisulfitlauge gewaschen werden, um überschüssiges Formaldehyd zu entfernen.

Variante B:

Allgemeine Synthesevorschrift:

Analog Variante A, jedoch wird das nach dem Umkristallisieren erhaltene Öl mit Hexan gewaschen, um überschüssiges Amid zu entfernen.

Variante C:

Allgemeine Synthesevorschrift:

0,25 mol Azol (II) werden, in 20 ml Methanol gelöst, vorgelegt und 0,1 mol 37 %ige wässrige Formaldehydlösung zugetropft. Man rührt noch eine halbe Stunde nach und tropft dann 0,125 mol Amid (IV), in 30 bis 50 ml Wasser gelöst, zu. Man erhitzt zwei bis acht Stunden unter Rückfluss und verfolgt die Reaktion per Dünnschichtchromatographie. Man lässt das Reaktionsgemisch abkühlen, verdampft das Lösungsmittel unter Vakuum und kristallisiert den erhaltenen Rückstand aus Wasser um.

Variante D:

Allgemeine Synthesevorschrift:

Analog Variante C, jedoch wird Wasser als Lösungsmittel verwendet.

Variante E:

Allgemeine Synthesevorschrift:

0,01 mol Hydroxymethyl-azol (V) und 0,01 mol Amid (IV) werden mit 5 ml Perfluoroctan (PF5080) vermischt und 0,1 g saurer Ionenaustauscher (DOWEX 50) sowie 2,5 g Molsieb 3A zugegeben. Es wird eine Stunde unter Rückfluss erhitzt. Das Perfluoroctan wird abdekantiert und der Reaktionsrückstand in 100 ml Chloroform aufgenommen. Molsieb und Ionenaustauscher werden durch Filtration abgetrennt. Die Chloroformlösung wird mit Magnesiumsulfat getrocknet, filtriert und das Lösungsmittel unter Vakuum entfernt. Gegebenenfalls wird umkristaliisiert.

Die in Tabelle 1 aufgeführten Substanzen wurden nach obigen Synthesevarianten erhalten. Die Strukturen wurden durch IR-, NMR-Spektroskopie und Elementaranalyse gesichert.

<u>Tabelle 1:</u> N-(1H-Azolyl-methyl)amide der Formel (I)

| Nr. | A | B | $R^1$ | $R^2$ | $R^3$ | $R^4$ | Ausb. (%) | Fp (°C)[1] | Variante | Elementaranalyse | | | |
|---|---|---|---|---|---|---|---|---|---|---|---|---|---|
| | | | | | | | | | | | C | H | N |
| 1 | N | O | H | H | H | Ph | 73 | 163-5[2] | | | - | - | - |
| 2 | CCl | O | $CH_3$ | H | H | Ph | 82 | 104-112 | A | ber.<br>gef. | 57,7<br>57,9 | 4,84<br>4,75 | 16,8<br>16,7 |
| 3 | CCl | O | $CH_3$ | H | H | $CH_3$ | 37 | 95-110 | A | ber.<br>gef. | 44,8<br>44,6 | 5,37<br>5,10 | 22,4<br>22,5 |
| 4 | CH | O | $CH_3$ | H | H | Ph | 44 | 94-104 | B | ber.<br>gef. | 67,0<br>67,1 | 6,09<br>5,87 | 19,5<br>19,0 |
| 5 | N | s | H | H | H | $NH_2$ | 49 | 170-72 | C | ber.<br>gef. | 30,6<br>30,3 | 4,49<br>4,61 | 44,0<br>43,7 |
| 6 | N | O | H | H | H | $NH_2$ | 42 | 228-31 | A | ber.<br>gef. | 34,0<br>34,4 | 5,00<br>4,45 | 49,6<br>49,4 |
| 7 | CCl | O | $CH_3$ | H | H | $NH_2$ | 65 | 157-60 | E | ber.<br>gef. | 38,2<br>37,9 | 4,80<br>4,89 | 29,7<br>29,8 |
| 8 | CCl | S | $CH_3$ | H | H | $NH_2$ | 41 | 179-81 | C | ber.<br>gef. | 35,2<br>35,7 | 4,43<br>4,56 | 27,4<br>27,1 |

(fortgesetzt)

| Nr. | A | B | $R^1$ | $R^2$ | $R^3$ | $R^4$ | Ausb. (%) | Fp (°C)[1] | Variante | Elementaranalyse | | | |
|---|---|---|---|---|---|---|---|---|---|---|---|---|---|
| | | | | | | | | | | | C | H | N |
| 9 | CH | O | $CH_3$ | H | H | 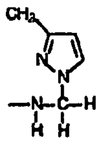 | 55 | 170-76 | C | ber.<br>gef. | 53,2<br>52,9 | 6,50<br>6,27 | 33,8<br>33,6 |
| 10 | CH | S | $CH_3$ | H | H | 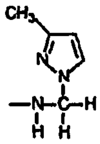 | 81 | 168-70 [3] | D | ber.<br>gef. | 50,0<br>50,1 | 6,10<br>6,28 | 31,8<br>31,8 |
| 11 | CH | S | H | H | $CH_2$-$CH_2$ | | 66 | 187-91 | C | ber.<br>gef. | 53,8<br>54,1 | 6,25<br>6,40 | 28,9<br>28,6 |

[1] Das Auftreten von Isomeren in Bezug auf die Stellung des $R^1$- und $R^2$-Substituenten zeigt sich gelegentlich durch etwas breitere Schmelzbereiche. Falls gewünscht, können die jeweiligen Isomeren mittels der üblichen, dem Fachmann bekannten Verfahren getrennt werden.
[2] A.R. Katritzky et al. Tetrahedron 46 (1990) 633 [3] SU 666174

Ermittlung der nitrifikationshemmenden Wirkung

Die Prüfung der erfindungsgemäßen N-(1H-Azolyl-methyl)amide erfolgte in den in Tabelle 2 ausgewiesenen Konzentrationen. Dabei wurde die nitrifikationshemmende Wirkung in der Weise ermittelt, dass 100 g lufttrockener Boden auf 50 % der maximalen Wasserkapazität eingestellt wurden. Hierzu wurden jeweils 10 mg Harnstoff-N und die ausgewiesene Wirkstoffmenge unter möglichst homogener Verteilung zugesetzt. Danach werden die so präparierten Gefäße unter standardisierten Bedingungen bei 20 °C im dunklen Klimaraum aufbewahrt. Aus der zeitabhängigen Bestimmung des Umsatzes von Ammonium-N zu Nitratstickstoff im Vergleich zur Variante ohne Inhibitor wird die prozentuale Hemmung zum Tag X ermittelt. Der nachfolgend verwendete K-Wert verkörpert einen von den biologischen Schwankungen der Bodenaktivität unabhängigen Wert, da er auf die Wirkung eines Standards, in diesem Fall 3-Methylpyrazol mit einer Inhibitorkonzentration von 0,2 % N-bezogen, durch Quotientenbildung relativiert wird. Der Ermittlung des K-Wertes ist die Berechnung des $t_{50}$ -Wertes vorangestellt. Dieser Wert drückt den Zeitpunkt in Tagen aus, an dem die Nitrifikationshemmung für eine einzelne Substanz auf 50 % zurückgegangen ist. Daraus leitet sich der K-Wert wie folgt ab:

$$K = t_{50}\text{–Wert der Testsubstanz} / t_{50}\text{–Wert Standard}$$

Tabelle 2: Nitrifikationshemmende Wirkung ausgewählter Verbindungen

| Nr. der Verbindung | Wirkstoffkonzentration ppm | K-Wert |
|---|---|---|
| 1 | 0,5<br>1,0 | 0,6<br>0,8 |
| 2 | 0,2<br>0,5 | 1,3<br>1,4 |
| 3 | 0,2<br>0,5 | 1,4<br>1,4 |
| 4 | 0,2 | 0,8 |
| | 0,5 | 1,1 |
| 5 | 1,0 | 0,7 |

(fortgesetzt)

| Nr. der Verbindung | Wirkstoffkonzentration ppm | K-Wert |
|---|---|---|
| 7 | 0,5<br>1,0 | 0,7<br>1,3 |
| 8 | 0,2<br>0,5 | 1,1<br>1,4 |
| 9 | 1,0 | 0,5 |
| 10 | 0,2<br>0,5 | 0,8<br>1,2 |
| 11 | 0,5<br>1,0 | 1,0<br>1,4 |

K-Werte um den Faktor 1 entsprechen dabei der Wirkung der Standardsubstanz. Unter diesem Aspekt kann Tabelle 2 entnommen werden, dass die erfindungsgemäßen Verbindungen über eine ausgezeichnete nitrifikationshemmende Wirkung verfügen, ohne die für eine praktische Anwendung entgegenstehenden Nachteile des Standards 3-Methylpyrazol wie Flüchtigkeit und Formulierungsunverträglichkeit mit speziell Feststoffdüngern aufzuweisen.

## Patentansprüche

**1.** N-(1H-Azolyl-methyl)amide der allgemeinen Formel I, **dadurch gekennzeichnet, dass**

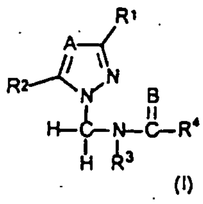

(I)

A = CH, C-Halogen, C-$C_1$-$C_4$-Alkyl oder C-$C_3$-$C_8$-Cycloalkyl oder N ist,
$R^1$ und $R^2$ unabhängig voneinander für H, $C_1$-$C_4$-Alkyl oder $C_3$-$C_8$-Cycloalkyl steht
$R^3$ für $C_1$-$C_4$-Alkyl, $C_3$-$C_8$-Cycloalkyl oder $C_6$-$C_{10}$-Aryl oder H, steht
$R^4$ für $C_1$-$C_{20}$-Alkyl, $C_3$-$C_8$-Cyclalkyl, $C_6$-$C_{10}$-Aryl oder Arylalkyl mit $C_1$-$C_4$-Alkyl- und $C_6$-$C_{10}$-Arylgruppen, wobei die Arylreste von $R^4$ gegebenenfalls einfach oder mehrfach mit $C_1$-$C_4$-Alkylsulfonyl, $C_1$-$C_4$-Alkoxy, $C_1$-$C_4$-Acyl, Halogen, Hydroxyl, Cyano, Nitro, Carboxyl oder $C_1$-$C_5$-Carboxyalkyl substituiert sein können, oder für $NHR^3$
oder für

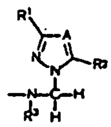

mit den für $R^1$, $R^2$, $R^3$ und A genannten Resten steht,
oder wobei $R^3$ und $R^4$ gemeinsam eine Ethylenbrücke-bilden können
und B für Sauerstoff oder Schwefel steht, wobei A nicht N sein darf, wenn $R^1$, $R^2$ und $R^3$ für H und $R^4$ gleichzeitig für Phenyl oder für - CH = CH - Phenyl steht, A nicht CH sein darf,
wenn $R^1$ für Methyl, $R^2$ und $R^3$ für H, B für Schwefel und $R^4$ für

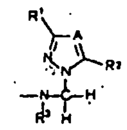

steht. und A nicht N sein darf, wenn $R^1$, $R^2$ für Methyl, $R^3$ für H, B für O und $R^4$ gleichzeitig für mit Halogen substituiertes Phenyl steht, als Mittel zur Hemmung bzw. Regelung der Nitrifikation.

**2.** N-(1H-Azolyl-methyl)amide nach Anspruch 1, **dadurch gekennzeichnet, dass** unabhängig voneinander $R^1$ einen Methyl- und A einen C-Methylrest darstellt und, vorzugsweise, $R^2$ H ist.

**3.** N-(1H-Azolyl-methyl)amide nach Anspruch 1, **dadurch gekennzeichnet, dass** $R^1$ einen Methylrest darstellt und A für C-Chlor oder CH steht und, vorzugsweise, $R^2$ H ist.

**4.** Verfahren zur Herstellung der N-(1H-Azolyl-methyl)amide 1 nach den Ansprüchen 1 bis 3, **dadurch gekennzeichnet, dass** man die entsprechenden Azole der allgemeinen Formel II

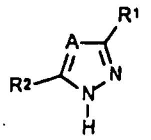

(II)

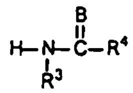

(IV)

wobei $R^1$, $R^2$ und A oben genannte Bedeutung besitzen, mit Formaldehyd und Amiden der allgemeinen Formel IV, wobei $R^3$, $R^4$ und B die oben genannte Bedeutung besitzen, reagieren lässt.

**5.** Verfahren nach Anspruch 4, **dadurch gekennzeichnet, dass** man die Reaktion bei Temperaturen von 100 bis 200 °C ohne Lösungsmittel in der Schmelze und mit einem Molverhältnis von Azol II zu Formaldehyd und Amid IV von 1 : 1 : 1 bis 2,5 : 3 : 1 durchführt.

**6.** Verfahren nach Anspruch 5, **dadurch gekennzeichnet, dass** man die Reaktion bei 125 bis 145 °C durchführt.

**7.** Verfahren nach Anspruch 4, **dadurch gekennzeichnet, dass** man die Reaktion bei Temperaturen von 40 bis 110 °C in Wasser als Lösungsmittel und mit einem Molverhältnis von Azol II zu Formaldehyd und Amid IV von 1 : 1 : 1 bis 2,5 : 3 : I durchführt.

**8.** Verfahren nach Anspruch 7, **dadurch gekennzeichnet, dass** man einen Lösungsvermittler aus der Gruppe der Alkohole oder Acetonitril zusetzt.

**9.** Verfahren zur Herstellung der N-(1H-Azolyl-methyl)amide I nach den Ansprüchen 1 bis 3, **dadurch gekennzeichnet, dass** man die entsprechenden 1-Hydroxymethyl-azole der allgemeinen Formel V

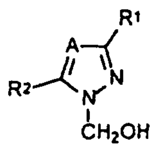

(V)

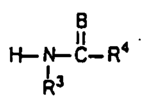

(IV)

wobei $R^1$, $R^2$ A oben genannte Bedeutung besitzen, mit Amiden der allgemeinen Formel IV, wobei $R^3$, $R^4$ und B die oben genannte Bedeutung besitzen, reagieren lässt.

**10.** Verfahren nach Anspruch 9, **dadurch gekennzeichnet, dass** man die Reaktion unter Zusatz von 0,2 bis 10 % einer geeigneten Säure, vorzugsweise eines saueren Ionenaustauschers, durchfürhrt.

**11.** Verfahren nach Anspruch 9, **dadurch gekennzeichnet, dass** die Reaktion ohne Lösungsmittel durchgeführt wird.

**12.** Verwendung von N-(1H-Azolyl-methyl)amiden entsprechend der allgemeinen Formel (I)

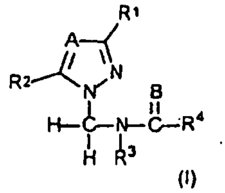

(I)

wobei A = CH, C-Halogen, C-$C_1$-$C_4$-Alkyl oder C-$C_3$-$C_8$-Cycloalkyl oder N ist,
$R^1$ und $R^2$ unabhängig voneinander für H, $C_1$-$C_4$-Alkyl oder $C_3$-$C_8$-Cycloalkyl steht
$R^3$ für $C_1$-$C_4$-Alkyl, $C_3$-$C_8$-Cycloalkyl oder $C_6$-$C_{10}$-Aryl oder H, steht
$R^4$ für $C_1$-$C_{20}$-Alkyl, $C_3$-$C_8$-Cycloalky), $C_6$-$C_{10}$-Aryl oder Arylalkyl mit $C_1$-$C_4$-Alkyl- und $C_6$-$C_{10}$-Arylgruppen, wobei die Arylreste von $R^4$ gegebenenfalls einfach oder mehrfach mit $C_1$-$C_4$-Alkylsulfonyl, $C_1$-$C_4$-Alkoxy, $C_1$-$C_4$-Acyl, Halogen, Hydroxyl, Cyano, Nitro, Carbonyl oder $C_1$-$C_5$-Carboxyalkyl substituiert sein können, oder für $NHR^3$ oder für

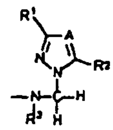

mit den für $R^1$, $R^2$, $R^3$ und A genannten Resten steht,
oder wobei $R^3$ und $R^4$ gemeinsam eine Ethylenbrücke bilden können und B für Sauerstoff oder Schwefel steht, als Mittel zur Hemmung bzw. Steuerung der Nitrifikation.

**13.** Zusammensetzung, aufweisend die N-(1H-Azolyl-methyl)amide nach den Ansprüchen 1 bis 3 sowie gegebenenfalls weitere Nitrifikationsinhibitoren und/oder Ureaseinhibitoren in Kombination mit festen und flüssigen ammonium- und/oder amidhaltigen Düngemitteln.

**14.** Zusammensetzung nach Anspruch 13, **dadurch gekennzeichnet, dass** die N-(IH-Azolyl-methyl)amide sowie die gegebenenfalls weiteren Nitrifikationsinhibitoren und/oder Ureaseinhibitoren in einer Gesamtaufwandmenge von 0,01 bis 10,0 Gewichtsprozent, berechnet auf den reduzierten Stickstoffanteil des amid- und/oder ammoniumhaltigen Düngemittels, eingesetzt werden.

**15.** Zusammensetzung nach Anspruch 14, **dadurch gekennzeichnet, dass** die N-(1H-Azolyl-methyl)amide in einer Aufwandmenge von 0,1 bis 3,0 Gewichtsprozent eingesetzt werden.

**16.** Zusammensetzung nach den Ansprüchen 13 bis 15, **dadurch gekennzeichnet, dass** die N-(1H-Azolyl-methyl) amide allein oder im Gemisch mit weiteren Zusatzstoffen in die Düngemittelschmelze oder -slurry während des Formgebungsprozesses eingebracht worden sind und auf diese Weise homogen im Dünger verteilt vorliegen.

**17.** Zusammensetzung nach den Ansprüchen 13 bis 15, **dadurch gekennzeichnet, dass** die N-(1H-Azolyl-methyl) amide ggf. mit weiteren Zusatzstoffen in Form einer Lösung oder gemeinsam mit einem geeigneten Konditionierungsmittel auf die Oberfläche der Düngemittelgranulate aufgebracht worden sind und gegebenenfalls anschließend das Lösungsmittel durch Trocknung entfernt worden ist.

## Claims

**1.** N-(1H-azolyl-methyl)amides of general formula I, **characterised in that**

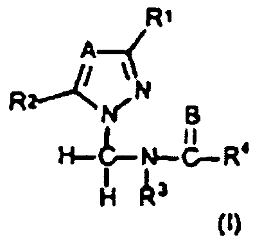

(I)

A=CH, C-halogen, C-$C_1$-$C_4$-alkyl or C-$C_3$-$C_8$-cycloalkyl or N,
$R^1$ and $R^2$, independently of one another, represent H, $C_1$-$C_4$-alkyl or $C_3$-$C_8$-cycloalkyl,
$R^3$ represents $C_1$-$C_4$-Alkyl, $C_3$-$C_8$-cycloalkyl or $C_6$-$C_{10}$-aryl or H,
$R^4$ represents $C_1$-$C_{20}$-alkyl, $C_3$-$C_8$-cydoalkyl, $C_6$-$C_{10}$-aryl or aryl alkyl with $C_1$-$C_4$-alkyl- and $C_6$-$C_{10}$-aryl groups, the aryl radicals of $R^4$ optionally being substituted one or more times by $C_1$-$C_4$-alkylsulfonyl, $C_1$-$C_4$-alkoxy, $C_1$-$C_4$-acyl, halogen, hydroxyl, cyano, nitro, carboxyl or $C_1$-$C_5$-carboxyalkyl, or represents $NHR^3$
or represents

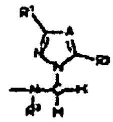

with the radicals mentioned for $R^1$, $R^2$, $R^3$ and A,
or in which $R^3$ and $R^4$ can together form an ethylene bridge
and B represents oxygen or sulphur, wherein A may not be N if $R^1$, $R^2$ and $R^3$ represent H and $R^4$ simultaneously represents phenyl or -CH=CH-phenyl, A may not be CH if R' represents methyl, $R^2$ and $R^3$ represent H, B represents sulphur and $R^4$ represents

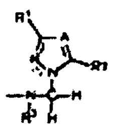

,

and A may not be N if $R^1$ and $R^2$ represent methyl, $R^3$ represents H, B represents O and $R^4$ simultaneously represents halogen-substituted phenyl, as agents for inhibiting or regulating nitrification.

2. N-(1H-azolyl-methyl)amides according to claim 1, **characterised in that**, independently of one another, $R^1$ represents a methyl radical and A represents a C-methyl radical, and, preferably, $R^2$ is H.

3. N-(1H-azolyl-methyl)amides according to claim 1, **characterised in that** $R^1$ represents a methyl radical and A represents C-chlorine or CH, and, preferably, $R^2$ is H.

4. Process for producing the N-(1H-azolyl-methyl)amides I according to claims 1 to 3, **characterised in that** the corresponding azoles of general formula II,

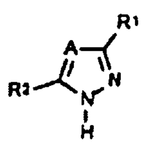

(II)

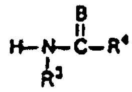

(IV)

in which $R^1$, $R^2$, and A have the meaning given above, are reacted with formaldehyde and amides of general formula IV, $R^3$, $R^4$ and B having the meaning given above.

5. Process according to claim 4, **characterised in that** the reaction is carried out at temperatures of from 100 to 200

°C without a solvent in the molten mass and with a molar ratio of azole II to formaldehyde and amide IV of from 1 : 1 : 1 to 2.5 : 3 : 1.

**6.** Process according to claim 5, **characterised in that** the reaction is carried out at 125 to 145 °C.

**7.** Process according to claim 4, **characterised in that** the reaction is carried out at temperatures of 40 to 110 °C in water as a solvent and with a molar ratio of azole II to formaldehyde and amide IV of from 1 : 1 : 1 to 2.5 : 3 : 1.

**8.** Process according to claim 7, **characterised in that** a solubiser from the group consisting of alcohols or acetonitriles is added.

**9.** Process for producing the N-(1H-azolyl-methyl)amides I according to claims 1 to 3, **characterised in that** the corresponding 1-hydroxymethyl-azoles of general formula V,

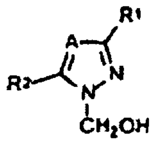

(V)

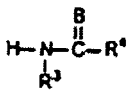

(IV)

in which $R^1$, $R^2$ and A have the meaning given above, are reacted with amides of general formula IV, in which $R^3$, $R^4$ and B have the meaning given above.

**10.** Process according to claim 9, **characterised in that** the reaction is carried out while adding from 0.2 to 10 % of a suitable acid, preferably an acidic ion exchanger.

**11.** Process according to claim 9, **characterised in that** the reaction is carried out without a solvent.

**12.** Use of N-(1H-azolyl-methyl)amides corresponding to the general formula (I),

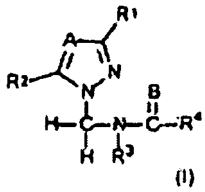

(I)

in which A=CH, C-halogen, C-$C_1$-$C_4$-alkyl or C-$C_3$-$C_8$-cyclohexyl or N,
$R^1$ and $R^2$, independently of one another, represent H, $C_1$-$C_4$-alkyl or $C_3$-$C_8$-cycloalkyl,
$R^3$ represents $C_1$-$C_4$-Alkyl, $C_3$-$C_8$-cycloalkyl or $C_6$-$C_{10}$-aryl or H,
$R^4$ represents $C_1$-$C_{20}$-alkyl, $C_3$-$C_8$-cycloalkyl, $C_6$-$C_{10}$-aryl or aryl alkyl with $C_1$-$C_4$-alkyl- and $C_6$-$C_{10}$-aryl groups, the aryl radicals of $R^4$ optionally being substituted one or more times by $C_1$-$C_4$-alkylsulfonyl, $C_1$-$C_4$-alkoxy, $C_1$-$C_4$-acyl, halogen, hydroxyl, cyano, nitro, carboxyl or $C_1$-$C_5$-carboxyalkyl, or represents $NHR^3$
or represents

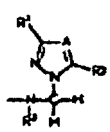

with the radicals mentioned for $R^1$, $R^2$, $R^3$ and A,
or in which $R^3$ and $R^4$ can together form an ethylene bridge
and B represents oxygen or sulphur,
as agents for inhibiting or controlling nitrification.

**13.** Composition, comprising the N-(1H-azolyl-methyl)amides according to claims 1 to 3, and optionally further nitrification inhibitors and/or urea inhibitors in combination with solid and liquid ammonium- and/or amide-containing fertilisers.

**14.** Composition according to claim 13, **characterised in that** the N-(1H-azolylmethyl)amides and the optional further nitrification inhibitors and/or urea inhibitors are used in a total amount of from 0.01 to 10.0 per cent by weight, based on the reduced nitrogen content of the amide- and/or ammonium-containing fertiliser.

**15.** Composition according to claim 14, **characterised in that** the N-(1H-azolylmethyl)amides are added in an amount of from 0.1 to 3.0 per cent by weight.

**16.** Composition according to claims 13 to 15, **characterised in that** the N-(1H-azolylmethyl)amides are introduced into the fertiliser molten mass or fertiliser slurry alone, or in the mixture with further additives, during the shaping process and in this manner are distributed uniformly in the fertiliser.

**17.** Composition according to any of claims 13 to 15, **characterised in that** the N-(1H-azolyl-methyl)amides have been applied, optionally with further additives in the form of a solution or together with a suitable conditioning agent, to the surface of the fertiliser granules, and the solvent has then optionally been removed by drying.

## Revendications

**1.** N-(1H-azolyl-méthyl)amides de formule générale I, **caractérisés en ce que**

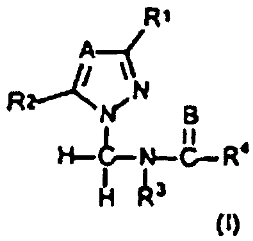

(I)

A = CH, C-halogène, C-$C_1$-$C_4$-alkyle ou C-$C_3$-$C_8$-cycloalkyle ou N,
$R^1$ et $R^2$ désignent, indépendamment l'un de l'autre, H, $C_1$-$C_4$-alkyle ou $C_3$-$C_8$-cycloalkyle,
$R^3$ désigne $C_1$-$C_4$-alkyle, $C_3$-$C_8$-cycloalkyle ou $C_6$-$C_{10}$-aryle ou H,
$R^4$ désigne $C_1$-$C_{20}$-alkyle, $C_3$-$C_8$-cycloalkyle, $C_6$-$C_{10}$-aryle ou arylalkyle avec des groupes $C_1$-$C_4$-alkyle et $C_6$-$C_{10}$-aryle, les radicaux aryle de $R^4$ pouvant éventuellement être substitués de façon unique ou multiple par $C_1$-$C_4$-alkylsulfonyle, $C_1$-$C_4$-alcoxy, $C_1$-$C_4$-acyle, halogène, hydroxyle, cyano, nitro, carboxyle ou $C_1$-$C_5$-carboxyalkyle, ou désigne $NHR^3$
ou

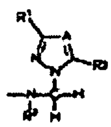

avec les radicaux cités pour $R^1$, $R^2$, $R^3$ et A,
ou $R^3$ et $R^4$ pouvant former en commun un pont éthylène
et B désigne l'oxygène ou le soufre, A ne devant pas être N si $R^1$, $R^2$ et $R^3$ désignent H et $R^4$ désigne le phényle ou - CH = CH - phényle, A ne devant pas être CH
si $R^1$ désigne le méthyle, $R^2$ et $R^3$ désignent H, B désigne le soufre et $R^4$ désigne

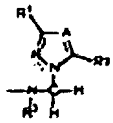

,

et A ne devant pas être N si $R^1$, $R^2$ désignent le méthyle, $R^3$ désigne H, B désigne O et $R^4$ a simultanément pour formule le phényle substitué par un halogène,
en tant que moyen pour l'inhibition ou la régulation de la nitrification.

**2.** N-(1H-azolyl-méthyl)amides selon la revendication 1, **caractérisés en ce que**, indépendamment l'un de l'autre, $R^1$ représente un radical méthyle et A un radical C-méthyle, et **en ce que**, de préférence, $R^2$ est H.

**3.** N-(1H-azolyl-méthyl)amides selon la revendication 1, **caractérisés en ce que** $R^1$ représente un radical méthyle et A désigne C-chlore ou CH, et de préférence, $R^2$ est H.

**4.** Procédé de production des N-(1H-azolyl-méthyl)amides I selon les revendications 1 à 3, caractérisés en que l'on fait réagir les azoles correspondants de formule générale II

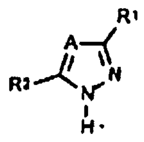
(II)

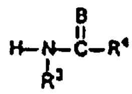
(IV)

dans laquelle $R^1$, $R^2$ et A ont la signification citée plus haut, avec du formaldéhyde et des amides de formule générale IV, dans laquelle $R^3$, $R^4$ et B ont la signification citée plus haut.

**5.** Procédé selon la revendication 4, **caractérisé en ce que** l'on effectue la réaction en présence de températures de 100 à 200 °C sans solvant dans la masse fondue et avec un rapport molaire de l'azole II au formaldéhyde et à l'amide IV de 1 : 1 : 1 à 2,5 : 3 : 1.

**6.** Procédé selon la revendication 5, **caractérisé en ce que** l'on effectue la réaction entre 125 et 145 °C.

**7.** Procédé selon la revendication 4, **caractérisé en ce que** l'on effectue la réaction en présence de températures de 40 à 110 °C dans de l'eau en tant que solvant et avec un rapport molaire de l'azole II au formaldéhyde et à l'amide IV de 1 : 1 : 1 à 2,5 : 3 : 1.

**8.** Procédé selon la revendication 7, **caractérisé en ce que** l'on ajoute un agent de solubilisation issu du groupe des alcools ou de l'acétonitrile.

**9.** Procédé de production des N-(1H-azolyl-méthyl)amides I selon les revendications 1 à 3, **caractérisé en ce que** l'on fait réagir les 1-hydroxyméthyl-azoles correspondants de formule générale V

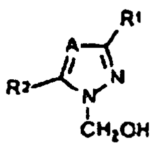
(V)

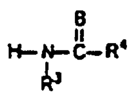
(IV)

dans laquelle $R^1$, $R^2$ et A ont la signification citée plus haut, avec des amides de formule générale IV, dans laquelle $R^3$, $R^4$ et B ont la signification citée plus haut.

**10.** Procédé selon la revendication 9, **caractérisé en ce que** l'on effectue la réaction en ajoutant 0,2 à 10% d'un acide approprié, de préférence un échangeur d'ions acide.

**11.** Procédé selon la revendication 9, **caractérisé en ce que** la réaction est effectuée sans solvant.

**12.** Utilisation de N-(1H-azolyl-méthyl)amides correspondant à la formule générale (I)

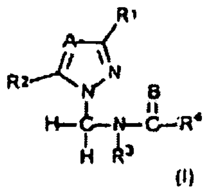

(I)

dans laquelle A = CH, C-halogène, C-$C_1$-$C_4$-alkyle ou C-$C_3$-$C_8$-cycloalkyle ou N,
$R^1$ et $R^2$ désignent, indépendamment l'un de l'autre, H, $C_1$-$C_4$-alkyle ou $C_3$-$C_8$-cycloalkyle,
$R^3$ désigne $C_1$-$C_4$-alkyle, $C_3$-$C_8$-cycloalkyle ou $C_6$-$C_{10}$-aryle ou H,
$R^4$ désigne $C_1$-$C_{20}$-alkyle, $C_3$-$C_8$-cycloalkyle, $C_6$-$C_{10}$-aryle ou arylalkyle avec des groupes $C_1$-$C_4$-alkyle et $C_6$-$C_{10}$-aryle, les radicaux aryle de $R^4$ pouvant éventuellement être substitués de façon unique ou multiple par $C_1$-$C_4$-alkylsulfonyle, $C_1$-$C_4$-alcoxy, $C_1$-$C_4$-acyle, halogène, hydroxyle, cyano, nitro, carboxyle ou $C_1$-$C_5$-carboxyalkyle, ou désigne $NHR^3$
ou

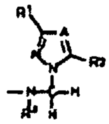

avec les radicaux cités pour $R^1$, $R^2$, $R^3$ et A,
ou $R^3$ et $R^4$ pouvant former en commun un pont éthylène
et B désigne l'oxygène ou le soufre,
en tant que moyen d'inhibition ou de régulation de la nitrification.

**13.** Composition, présentant les N-(1H-azolyl-méthyl)amides selon les revendications 1 à 3 ainsi éventuellement que d'autres inhibiteurs de la nitrification et/ou inhibiteurs de l'uréase en combinaison avec des engrais solides et liquides contenant de l'ammonium et/ou des amides.

**14.** Composition selon la revendication 13, **caractérisée en ce que** les N-(1H-azolylméthyl)amides ainsi que les éventuels autres inhibiteurs de la nitrification et/ou inhibiteurs de l'uréase sont utilisés à raison d'une quantité globale de 0,01 à 10,0 % en poids, par rapport à la fraction d'azote réduite de l'engrais contenant de l'ammonium et/ou des amides.

**15.** Composition selon la revendication 14, **caractérisée en ce que** les N-(1H-azolylméthyl)amides sont utilisés à raison d'une quantité de 0,1 à 3,0 % en poids.

**16.** Composition selon les revendications 13 à 15, **caractérisée en ce que** les N-(1H-azolyl-méthyl)amides ont été introduits dans la masse fondue d'engrais ou la bouillie d'engrais, seuls ou dans le mélange avec d'autres additifs, pendant le processus de façonnage et sont de cette façon répartis de façon homogène dans l'engrais.

**17.** Composition selon les revendications 13 à 15, **caractérisée en ce que** les N-(1H-azolyl-méthyl)amides ont été appliqués sur la surface des granulés d'engrais éventuellement avec d'autres additifs sous forme de solution ou en commun avec un moyen de conditionnement approprié, et **en ce que** le solvant a ensuite éventuellement été éliminé par séchage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 3635690 A
- US 4969946 A
- US 4523940 A
- EP 166420 A
- JP OS7104135 A
- US 3697244 A
- DE OS2745833 A
- EP 508191 A
- DD 292232
- DE OS4018395 A
- EP 160804 A
- DE OS3704359 A
- SU 1470737
- DE OS4446194 A
- EP 808297 A
- EP 529473 A
- WO 9805607 A
- DE 3704359 A
- SU 1470737 A
- DE OS10117821 A
- DE OS10135423 A
- EP 1342412 A
- DE 2648008 A
- EP 0012216 A
- EP 0006542 A
- DE 2742583 A
- SU 666174 A
- DE 19822340 A1

### In der Beschreibung aufgeführte Nicht-Patentliteratur

- **KATRITZKY et al.** *Tetrahedron,* 1990, vol. 46 (2), 633-40
- *J. Hüttel Chem. Ber.,* 1952, vol. 85, 820
- **L. MAIER.** *Phosphorus Sulfur,* 1987, vol. 33, 41